(19) Europäisches Patentamt
European Patent Office
Office européen des brevets

(11) **EP 2 098 128 A1**

(12) **EUROPEAN PATENT APPLICATION**

(43) Date of publication:
**09.09.2009 Bulletin 2009/37**

(21) Application number: **08102399.6**

(22) Date of filing: **07.03.2008**

(51) Int Cl.:
*A23L 1/30* (2006.01)    *A23L 1/308* (2006.01)
*A23L 1/09* (2006.01)    *A23L 2/52* (2006.01)
*A23L 1/054* (2006.01)    *C12N 9/10* (2006.01)
*C12N 9/24* (2006.01)    *C12P 19/04* (2006.01)
*C12P 19/18* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Bayer CropScience AG**
**40789 Monheim (DE)**

(72) Inventors:
• **Pilling, Jens**
  **44287 Dortmund (DE)**
• **Frohberg, Claus**
  **14532 Kleinmachnow (DE)**

(74) Representative: **Beyer, Andreas**
**Bayer BioScience GmbH**
**Hermannswerder 20a**
**14473 Potsdam (DE)**

(54) **The use of alternan as a heat-stable ingredient for a foodstuff**

(57)    The invention relates to the use of alternan as a heat stable ingredient in a foodstuff, a method for manufacturing a foodstuff, comprising the addition of alternan as an ingredient to the foodstuff formulation and a heating step, and to a foodstuff comprising alternan as ingredient, wherein the foodstuff was subjected to a heating step during its manufacture.

EP 2 098 128 A1

**Description**

The present invention relates to the use of alternan as a heat stable ingredient in a foodstuff, a method for manufacturing a foodstuff, comprising the addition of alternan as an ingredient to the foodstuff formulation and a heating step, and to a foodstuff comprising alternan as ingredient, wherein the foodstuff was subjected to a heating step during its manufacture.

Many nutritional ingredients, as dietary fibers, are heat sensitive. Dietary fiber is an important component of the diet but many consumers find dietary fibers unpalatable. Some dietary fibers, as resistant starches (RS), which many consumers find more palatable, do not retain their high dietary fiber content under harsh processing conditions, resulting in products with less dietary fiber than theoretically anticipated. Many foods are subjected to harsh processing conditions, such as homogenization of high moisture food formulations including puddings and yogurts and further pasteurization at temperature 70°C or higher, retorting where temperature is at 121°C for prolonged period of time, and/or extrusion of low moisture food products including snacks and breakfast cereals. As harsh processing is used to produce a number of common food compositions, this has been seen as a major impediment to the adoption and use of dietary fibers in such processed food compositions.

US20070275123A1 suggests the use of a modified starch to increase the dietary fiber content of food compositions processed under harsh conditions. By using certain modified starches, food formulations may be harshly processed while retaining substantial dietary fiber. Further, modified starches as described in US20070275123 Al provide dietary fiber without the negative effects on textural or organoleptic properties of the food compositions which are typically associated with the addition of other dietary fiber sources.

In order to keep the total dietary fiber content high, alternative sources of fiber have been used. However, there is still a demand for heat stable functional ingredients for nutritional purposes.

Surprisingly, it has been found in the present invention that by using alternan as an ingredient, food compositions may be subjected to harsher processing conditions while avoiding a degradation of the ingredient.

The present invention is directed to the use of alteman as a heat stable ingredient in a foodstuff formulation.

The present invention is further directed to the use of alteman as a degradation-resistant ingredient in a manufacturing method for a foodstuff and to a method for manufacturing a foodstuff, comprising the addition of alteman as an ingredient to a foodstuff formulation and a heating step.

Alternan is deemed to have prebiotic properties (Lopez-Munguia et al Enzyme Microb, Technol. 15 (1993)). Other beneficial properties have been described. According to US 5,702,942 and US 5,789,209 altemans have unique properties that resemble certain functional characteristics of gum arabic, maltodextrins or polydextrose (G. L. Cote, Carbohydrate Polymers 19:249-252 (1992)). Moreover, according to US 5,702,942 and US 5,789,209 alteman has potential commercial applications as a low-viscosity bulking agent and extender in foods and cosmetics, so that altemans have value as noncaloric, carbohydrate-based soluble dietary fiber.

In the present invention it is shown that alteman is not degraded in under high temperature and could therefore retain the above-mentioned properties when used as a heat stable ingredient in a foodstuff formulation or in a manufacturing method for a foodstuff which comprises a heating step, respectively. In similar manner, the low glycemic properties and soluble fiber properties of altemans are retained since no glucose is released from the alternan-oligosaccharide molecules.

The term "alteman" according to the present invention encompasses alteman-polysaccharide and alteman-oligosaccharide, wherein both types of alteran differ in their molecular weights, as further defined below.

Alternan according to the present invention is a saccharide composed of glucose units. The glucose units are linked to each other via $\alpha$-1,3- and $\alpha$-1,6-glycosidic bonds, and said two types of bonds predominantly appear alternatingly. Alternan may contain branches (Seymour et al., Carbohydrate Research 74, (1979), 41-62).

Alternan-polysaccharide according to the definition of the present invention has a weight average molecular weight Mw of more than 3 000 g/mol, preferably more than 5 000 g/mol (determined with GPC RI or GPC MALLS). In another preferred embodiment, alteman-polysaccharide has a weight average molecular weight Mw in the range of 10 000 000 g/mol to 60 000 000 g/mol (determined with GPC MALLS), more preferably in the range of 12 000 000 g/mol to 50 000 000 g/mol. In a special embodiment, alteman-polysaccharide is produced with altemansucrase originating from Leuconostoc Mesenteroides as described in WO 00/47727 and shows a weight average molecular weight Mw in the range of 33 000 000 g/mol to 60 000 000 g/mol (determined with GPC MALLS), more preferably in the range of 33 000 000 g/mol to 50 000 000 g/mol. In still another special embodiment, alteman-polysaccharide is produced with truncated altemansucrase enzyme as described in PCT/EP2008/051760 and shows a weight average molecular weight Mw in the range of 12 000 000 g/mol to 30 000 000 g/mol (GPC MALLS), more preferably in the range of 14 000 000 g/mol to 28 000 000 g/mol, still more preferably in the range of 16 000 000 g/mol to 26 000 000 g/mol, most preferably 19 000 000 g/mol to 23 000 000 g/mol. Truncated altemansucrase enzymes, methods for producing alteman-polysaccharide therefrom as well as the alteman-polysaccharide itself are described in PCT/EP2008/051760, which is incorporated herein by reference in its entirety.

The weight average molecular weight Mw of alteman-oligosaccharide in the definition of the present invention is 3 000 g/mol or below, preferably 2 500 g/mol or below, more preferably 2 000 g/mol or below, most preferably 1 500 g/mol or below (determined with GPC RI or GPC MALLS). Thus the weight average degree of polymerization DPw of alteman oligosaccharide according to the invention is 18 or below, preferably 15 or below, more preferably 12 or below, most preferably 9 or below (DPw = Mw/162 g/mol, 162 g/mol = molecular weight of monomeric glucose unit).

Alternan-oligosaccharide of the present invention predominantly consists of alteman molecules having a degree of polymerization in the range of 3 - 20, wherein minor amounts of molecules with a higher DP might be present. In a preferred embodiment, alteman-oligosaccharide according to the invention consists of molecules having a degree of polymerization (DP) in the range of 3 - 15, more preferably in the range of 3 - 12, still more preferably in the range of 3 - 10, and most preferably in the range of 3 - 8.

In the present invention the singular-term "alteman-polysaccharide" designates both monodisperse alteman-polysaccharides with molecules of only one degree of polymerization (DP) as well as polydisperse alteman-polysaccharides with molecules having different degrees of polymerization.

In the present invention the singular-term "alteman-oligosaccharide" designates both monodisperse alteman-oligosaccharides with molecules of only one degree of polymerization (DP) as well as polydisperse alteman-oligosaccharides with molecules having different degrees of polymerization.

Alternan-oligosaccharides are wellknown from the state of the art and for example disclosed together with manufacturing processes in WO 00/47727, US 7,182,954 WO 2006/088884, and Cote and Robyt 1982, Carbohydrate Research, 111:127-142 which are incorporated by reference in their entirety in the present description.

Alternan-oligosaccharides can be produced from alteman polysaccharides by degradation of alteman polysaccharides under appropriate conditions. The degradation can for example be an enzymatic degradation of alteman polysaccharides or a degradation under acidic conditions, preferably with heating.

Alternan-oligosaccharides can also be produced by the reaction of sucrose with an acceptor molecule in presence of altemansucrase enzyme, which is preferred according to the present invention.

Alternan-oligosaccharides may be prepared by a method, wherein

> a) a sucrose containing solution is contacted with a catalytically effective amount of altemansucrase enzyme and acceptor molecules under conditions permitting the conversion of sucrose to alteman-oligosaccharide and fructose; and
>
> b) alteman-oligosaccharide and fructose are isolated from the solution.

The reaction may be conducted between room temperature and 37°C and at a pH between about 5 and 7, and may be allowed to proceed until the sucrose has been essentially consumed. Detailed reaction conditions are disclosed in WO 00/47727, US 7,182,954 and in the appended example. The product is usually obtained as a syrup which may further be purified, i.e. by membrane filtration, and/or dried.

The acceptor molecule is understood to mean a molecule at which an altemansucrase is able to catalyze a chain-extending reaction. The acceptor which can be added to the reaction mixture at the beginning of the reaction is preferably a carbohydrate or a carbohydrate derivative. The use of external acceptors leads to the production of low molecular alteman-oligosaccharides. The carbohydrate acceptor is preferably a saccharide selected from the group consisting of maltose, isomaltose, maltitol, (iso)maltotriose and methyl-$\alpha$-D-glucan. Other preferred acceptor molecules are glucose, gentiobiose, raffinose, melibiose, isomaltitol, isomaltooligosaccharide, theanderose, kojibiose, glucosyl trehaloses, cellobiose, maltotetraose, nigerose, lactose, panose or mixtures thereof.

Depending upon the particular acceptor selected, the glucosyl units will generally be added through an $\alpha(1,6)$ linkage, or through an $\alpha(1,3)$ linkage if an $\alpha(1,6)$ linkage is already present. Alternan-oligosaccharides are obtained which have a lower molecular weight than the alteman that can be prepared in the absence of external acceptors. The reaction will typically produce a mixture of oligosaccharides having different degrees of polymerization (DP). If alteman-oligosaccharides are produced by the reaction of sucrose with an acceptor molecule, the degree of polymerization (DP) is defined as the number of D-glucosyl units added onto the original acceptor molecule plus the number of monosaccharide units in the original acceptor oligosaccharide.

The extent of the degree of polymerization may vary with the concentrations and the relative ratio of sucrose and acceptor oligosaccharide. The reaction product will generally be composed of a mixture of oligosaccharides having different degrees of polymerization. At a relatively high sucrose:acceptor ratio, more glucosyl units are transferred into glucan and products with higher degree of polymerization are obtained (i.e. the relative amounts of the high DP oligosaccharides in the product will be increased). In contrast, at a low sucrose:acceptor ratio, the predominant reaction product is that resulting from the transfer of a single glucosyl unit to the acceptor. Thus, the yields of oligosaccharides of a desired degree of polymerization may be optimized by varying the sucrose:acceptor ratio. The precise sucrose:acceptor ratios for a desired degree of polymerization will vary with the particular acceptor oligosaccharide and may be readily determined by routine experimentation.

Alternansucrase for use herein may be obtained from a variety of microorganisms, preferably strains of Leuconostoc and particularly strains of L. mesenteroides, as for example disclosed in WO 00/47727. In a preferred embodiment, the enzyme is produced by strains of which secrete a high proportion of alternansucrase to dextransucrase such as described by Leathers et al., U.S. Pat. No. 5,702,942, the contents of which are incorporated by reference herein. In another preferred embodiment the alternansucrase enzymes that can be used to produce alternan-oligosaccharides include Leuconostoc mesenteroides strains NRRL B 1355, 23185, 23186, 23188, 23311, 21297, 30821, 30894 These enzymes can be additionally cloned and expressed recombinantly, such as described in Gilles Joucla, Doctoral Dissertation, Ingenier INSA, Toulouse, France, 2003.

Production of the alternansucrase may be conducted by culture of any of the above-mentioned microorganisms using conventional techniques and under aerobic conditions which are effective to promote growth and production of the enzyme such as described in Leathers et al. or the example herein below. Following culture, the enzyme may be isolated or separated from the microorganisms using conventional techniques, such as by centrifugation or filtration.

The terms "heat stable" and "degradation-resistant" in the present invention mean that the degree of polymerization of alternan does not measurably (High performance anion-exchange chromatography - HPAEC or Gel permeation chromatography - GPC-RI) decrease when heated to a temperature of up to 120°C over a time period of 1 hour at pH 7.

A main product of degradation of alternan is glucose and the degradation mechanism normally takes place by hydrolysis of alternan chains. Because of its degradation-resistance, alternan is excellently suitable as a degradation-resistant prebiotic and/or low glycemic and/or soluble fiber ingredient for foodstuffs which are heated during their manufacture. Moreover, all other beneficial properties of alternan that have been described in the prior art are retained due its degradation resistance.

As stated above, the present invention is directed to the use of alternan as a heat stable ingredient in a foodstuff formulation. The foodstuff formulation can be subjected to a heating step during the manufacturing process of the foodstuff or even later, e.g. when the foodstuff is heated by the consumer before consumption. In the first sense, i.e. heating during the manufacturing process, the invention is also directed to a method for manufacturing a foodstuff, comprising the addition of alternan as an ingredient to a foodstuff formulation and a heating step. The manufacturing method is not further limited. According to the present invention, alternan can be used in any manufacturing method for foodstuffs which comprises a heating step.

In case of heating during manufacturing process of a foodstuff, the heating step, or at least one of several heating steps, is performed after addition of alternan to the foodstuff formulation. Otherwise alternan would not fulfill the function of a degradation-resistant ingredient in the sense of the present invention.

The heating step, in the manufacturing method or even later, is preferably carried out at a temperature of 50-150°C, more preferably 60-150°C or 75-150°C, still more preferably 80 - 130°C, most preferably 80 - 120°C. In another preferred embodiment, the heating step is carried out at 60 - 120°C.

The time period of heating is preferably 5 to 1800 seconds, particularly 5 - 300 seconds, more preferably 5 - 200 seconds, even more preferably 5 - 100 seconds, especially preferably 5 - 60 seconds and most preferably 10 - 30 seconds. Each of these time periods can be combined with each of the temperatures specified above.

A well known heating method is pasteurization which is commonly used in the manufacture of dairy products, milk, ice creams, beverages, beer, canned foods, sauces, and soups. A pasteurization step is usually conducted at a temperature of about 60°C to about 100°C, preferably at about 75°C to about 85°C, for a time of about 10 seconds to about 30 minutes, preferably for at least about 25 seconds. Preferably, pasteurization is conducted by either high temperature short time (HTST) or low temperature long time (LTLT) processing.

Other common heating methods are sterilization and ultra high temperature (UHT) processing. In the present invention said methods are performed as commonly known to an expert skilled in the art. A UHT treatment is commonly undertaken at a temperature of 90 - 150°C, more preferably 95 - 150°C, even more preferably 100 - 150°C, and most preferably at 110°C - 150°C and a UHT treatment period is commonly 5 - 300 seconds, more preferably 5 - 200 seconds, even more preferably 5 - 100 seconds, especially preferably 5 - 60 seconds and most preferably 10 - 30 seconds.

In another aspect, the present invention is directed to a foodstuff which was subjected to a heating step during its manufacture and which comprises alternan as ingredient. The heating methods as explained above can be applied. The term "foodstuff" as used herein also encompasses beverages.

The foodstuff is preferably selected from dairy products, ice creams, yogurts, milk, puddings, beverages, beer, sauces, soups, retorted foodstuffs, condiments, canned foodstuffs, especially canned fruits, canned fish, canned vegetables, bakery products, cookies, cake, biscuits, meat products, extrusion products, as snacks and cereals, pasta and ready to serve meals..

Alternan is preferably added to the foodstuff according to the invention in an amount of 0.1 - 20 weight-% based on the total weight of the foodstuff, more preferably in an amount of 0.1 - 10 weight-%, still more preferably in an amount of 0.1 - 5 weight-%.

In a very special embodiment of the present invention, the foodstuff is an acidic foodstuff, which was subjected to a heating step during its manufacture and which comprises alternan as ingredient. Many foodstuffs which are subjected to a heating step during manufacture have a low pH which further promotes the degradation of valuable ingredients. This is especially true for acidic beverages since beverages are often heated to higher temperatures during the process, e.g. during a hot filling process. Other acidic foodstuffs, as for example dairy products, are commonly subjected to a pasteurization step.

An acidic foodstuff is defined herein as a foodstuff having a pH below 7. The acidic foodstuff has preferably a pH of 6 or below, more preferably a pH of 3.5 or below. In still another preferred embodiments the acidic foodstuff has a pH in the range of 1 - 6, more preferably 1 - 5, still more preferably 1 - 4 and most preferably 1.5 - 3.5. Another advantageous pH range is pH 3 - 6.

Preferred acidic foodstuffs are selected from canned foodstuffs, especially canned fruits, canned fish, canned vegetables, bakery products, cake, ready to serve meals, dairy products, as yogurt and buttermilk, and acidic beverages.

In a highly preferred embodiment, the acidic foodstuff is an acidic beverage. The acidic beverage has preferably a pH of 6 or below, more preferably a pH of 3.5 or below. In still another preferred embodiments the acidic beverage has a pH in the range of 1 - 6, more preferably 1 - 5, still more preferably 1 - 4 and most preferably 1.5 - 3.5. Another advantageous pH range is pH 3 - 6. The acidic beverage is preferably selected from fruit juices, energy drinks, lemonades, sherbets, sodas, soft drinks, and flavored waters.

In the present invention it is shown that alteman polysaccharide is only degraded to a minor amount in aqueous environment of pH 3-4 when heated to a temperature of up to 60°C for 1 hour. Alternan polysaccharide is deemed to be substantially stable even at 70°C under these conditions. Thus, alternan-polymer is a suitable as a degradation-resistant ingredient for acidic foodstuffs with pH 3 as a lower pH limit, which are subjected to a heating step at a temperature of up to 60-70°C. Degradation is detected by increase of glucose (High performance anion-exchange chromatography - HPAEC or Gel permeation chromatography - GPC-RI) in a heated sample as degradation product of alteman-polymer.

In foodstuffs which are still more acidic, as for example some acidic beverages, the preferred ingredient is alternan-oligosaccharide. In the present invention it is shown that alteman-oligosaccharide is not measurably degraded in aqueous environment of pH 3 or above when heated to a temperature of up to 120°C for 1 hour. In aqueous environment of pH 1.5 alteman-oligosaccharide is not measurably degraded when heated to a temperature of up to 95°C for 1 hour. No measurable degradation in this context means that no increase of glucose is detected (High performance anion-exchange chromatography - HPAEC or Gel permeation chromatography - GPC-RI) in a heated sample as degradation product of alternan-oligomers.

This property of alteman-oligosaccharide is particularly and without limitation beneficial in the manufacturing process of a beverage since beverages are sometimes heated to higher temperatures during the process. Some beverage processes call for hot filling. This involves heating the beverage to 80-90°C, holding for about 10 minutes at that temperature, cooling to about 65°C and then bottling. The alteman-oligosaccharide can withstand this heat abuse for at least about 10 minutes without visible effects.

The beverage according to the invention is preferably a clear beverage. Besides its degradation resistance, a further beneficial property of alteman-oligosaccharide is its ability to retain the clarity of a clear beverage when it is added to a clear beverage formulation as a prebiotic and/or low glycemic and/or soluble fiber ingredient. Clarity can be determined using the test procedure described in WO2006/088884 or evaluated visually on a qualitative basis.

The following examples are merely intended to further illustrate the invention and are not intended to limit the scope of the invention which is defined by the claims.

**Examples**

Literature:

- Arguello-Morales MA, Remaud-Simeon M, Pizzut S, Sarcabal P, Willemot R and Monsan P (2000) Sequence analysis of the gene encoding altemansucrase, a sucrose glucosyltransferase from Leuconostoc mesenteroides NRRL B-1355. FEMS Microbiol Lett 182: 81-85.
- Degenkolb J, Takahashi M, Ellestad GA, Hillen W, 1991:Antimicrob. Agents Chemother. 35, No 8, 1591-1595 Structural requirements of tetracycline-Tet repressor interaction: Determination of equilibrium binding constants for tetracycline analogues with the Tet repressor.
- Dotto GP, Horiuchi K, Zinder ND (1982) Initiation and termination of phage f1 plusstrand synthesis. Proc Natl Acad Sci U S A. 79:7122-7126.
- Horn, U., Strittmatter, W., Krebber, A., Knüpfer, U., Kujau, M., Wenderoth, R., Müller, K., Matzku, S., Plückthun, A., and Riesenberg, D. (1996) High volumetric yields of functional dimeric miniantibodies in Escherichia coli, using an optimized expression vector and high-cell-density fermentation under non-limited growth conditins. Appl. Microbio. Biotechnol. 46, 524-532.
- Jeong SH, Bae IK, Lee JH, Sohn SG, Kang GH, Jeon GJ, Kim YH, Jeong BC and Lee SH. (2004) Molecular Characterization of Extended-Spectrum Beta-Lactamases Produced by Clinical Isolates of Klebsiella pneumoniae and Escherichia coli from a Korean Nationwide Survey. J Clin Microbiol 42: 2902-2906.

- López-Muguía, A., Pelenc, V., Remaud, M., Biton, J.1, Michel, J.M., Lang, C., Paul, F., Monsan, P. (1993). Production and purification of altemansucrase, a glucosyltransferase from Leuconostoc mesenteroides NRRL B-1355, for the synthesis of oligoalternan: Enzyme Microb. Technol., 15, 77-85.
- Schmidt TGM and Skerra A (1993). The random peptide library-assisted engineering of a C-terminal affinity peptide, useful for the detection and purification of a functional Ig Fv fragment. Protein Eng 6: 109-122.
- Skerra, A (1994). Use of the tetracycline promoter for the tightly regulated production of a murine antibody fragment in Escherichia coli. Gene 151, 131-135.

1. Fermentative production of alternansucrase from Leuconostoc mesenteroides

For heterologous expression of the altemansucrase (ARS) from Leuconostoc mesenteroides strain NRRL B-1355, the gene encoding altemansucrase has been isolated, fused to a Streptag (IBA BioTAGnology, Göttingen, Germany) and cloned into vector pAI-B-AlSu under the control of a tetrayclice-inducible promoter.

The genetically modified E. coli strain DH5a pAIB-AlSu Q29 employed for fermentation harbours plasmid pAI-B-AlSu for cytoplasmic expression of altemansucrase from Leuconostoc mesenteroides. Vector pAI-B-AlSu is essentially derived from plasmid pASK-IBA-3 (purchased from IBA Göttingen; www.iba-go.com). It contains the coding sequence of altemansucrase derived from Leuconostoc mesenteroides strain NRRL B-1355 fused to a 8 aminoacid peptide strep-tag at the C-terminal end. The strep-tag is linked to the protein through a 2 aminoacid linker. Expression of altemansucrase is under the transcriptional control of the tetA promoter/operator and repressor. The tetA promoter is tightly regulated by the tet repressor which is encoded on the same plasmid and is constitutively expressed from the β-lactamase promoter. In this way, expression of altemansucrase is stringently repressed until efficient chemical induction by tetracycline or anhydrotetracycline, AHT, (Degenkolb et al.; 1991).

Vector pAI-B-AlSu contains the following genetic elements:

| Nt Positions | Orientation | Origin |
|---|---|---|
| 37-72 | Clockwise | PTet: Tet Promoter from transposon Tn10 (Skerra, 1994) |
| 139-6309 | Clockwise | asrLm: Coding sequence of the Altemansucrase gene from *Leuconostoc mesenteroides* NRRL B-1355. (Arguello-Morales *et al.*, 2000) |
| 6310-6315 | Clockwise | SA linker encoding 2 Amino Acids: Serine and Alanine |
| 6316-6345 | Clockwise | StrepTag: sequence encoding a 8 AA peptide selected from a genetic fusion peptide library for its ability to bind to streptavidin (Schmidt and Skerra, 1993) |
| 6439-6877 | Clockwise | f1 origin: origin of replication from filamentous phage f1 (Dotto *et al.*, 1982) |
| 7026-7886 | Clockwise | AmpR: Coding sequence of β-lactamase from Escherichia coli, conferring Ampicillin resistance (Jeong *et al.*, 2004) |
| 7896-8519 | Clockwise | TetR: Tet repressor domain from Transposon Tn10 (Skerra, 1994) |

For fermentation, vector pAI-B-AlSu is transformed in E. coli K12 DH5α and bacterial cells harbouring the vector are selectively grown for 12 h at 37°C to an OD600 of 65 in mineral medium (Horn et al., 1996) supplemented with ampicillin (100 μg/ml). Expression of the alternansucrase is induced by the addition of anhydrotetracyclin (0.2 mg/1) and further cultivation for 5 h at 25°C to an OD600 of 140. For purification of the enzyme, the bacterial cells are harvested by centrifugation (20000 rpm; 20 min) and solubilized in resuspension buffer (100 mM NaAc, pH 5.3).

The cells are disrupted using a high pressure homogenizer (two cycles, 1200 bar). Bacterial nucleic acid is degraded by DNase/RNase (3 mg/1) treatment and the resulting extract is centrifuged (3,800 g for 15 min at 4°C) to harvest the insoluble cell matter including the bacterial inclusion bodies.

The supernatant is discarded and the pellet is resuspended 8 M urea, 50 mM NaAc buffer, pH 5.3 and kept on ice while shaking for one hour. Subsequently, the remaining debris is removed by centrifugation at 10.000 g for 15 min. Renaturation is then performed by 20-fold dilution in 0.5 M urea, 2.5 mM $CaCl_2$, 100 mM NaAc, pH 5.3. Aliquots of the mixture are immediately frozen in liquid nitrogen and stored at -20 °C.

Activity of alternansucrase can be determined as described by Lopez-Munguia et al., 1993.

2. Production of alternan-oligosaccharides with acceptor molecule maltose (maltose oligosaccharides)

For the production of maltose oligosaccharides, the purified alternansucrase enzyme as described in 1 is incubated with 10 % sucrose (w/v) and 4.5-5% maltose (w/v) in a 50 mM sodium acetate buffer pH 5.3 at room temperature for approximately 72 hours. For the incubation, 50 units of alternansucrase per litre reaction mix are used. Alternan, which accumulates as a by-product, is precipitated by adding analytical grade ethanol to a final concentration of 50 % (v/v). The mixture is centrifuged at 4000 rpm for 10 min and the resulting precipitate is discarded.

Maltose oligosaccharide is prepared by further concentrating the obtained precipitate using a vacuum vaporizer (Buechi Rotavapor R-220) to about 70° brix. Maltose oligosaccharide is obtained as a syrup which is further purified and dried to obtain the final product.

3. Production of alternan-polysaccharide

Plasmid pAI-B-AlSu Q29 (cf. Example 1 above) was transformed in E. coli DH5α. The cells were pre-cultured in mineralmedium (Horn et al., 1996) with 100 μg/ml Ampicillin and 10% LB medium. Mineralmedium, without LB, was inoculated with this pre-culture. The cells were grown at 37°C, induced with Anhydrotetracyclin (AHT) (0.2 mg/L), and grown further at 25°C. The cells were harvested, resuspended in [10 mM MOPS pH 7,6; 2,5 mM CaCl2 und 0,05 % Trition X-100] and extracted with a high pressure homogenisator. The cell lysate was centrifuged at 20 000 rpm for 20 minutes at 4°C. The supernatant was filtered over a 0.22 μm filter.

Alternan was produced in a 60 L Biotransformation containing 0.13% Acetic Acid, 100 mM NaAc pH5.3, 20% Sucrose, 1 mM DTT, 1600 ml filtered protein extract (ca. 3900 Units). The reaction mixture was incubated for 60h. at 37°C. The polymer was precipitated with 60L Technical Ethanol 40 h 4°C, washed 2x with 60 L 60% Technical Ethanol, and 1x with 60 L 60% Ethanol Absolute. The product was dried through lyophilization

4. Hot fill simulation in flavored waters

Two retail flavored waters were testes in an application study. Alternan-oligosaccharide (also designated hereinafter as "maltose oligosaccharide" because auf maltose acceptor) produced as described in Example 2 was used for the tests.

| Beverage Name | pH | Comments |
|---|---|---|
| Metro Mint® (Soma Beverage Co.) | 5.87 | Clear, colorless |
| Fruit2O® (Kraft) | 3.02 | Clear, colorless |

Set Up

- Storage Temperatures: -17.8°C, 4.4°C, 21°C, 32.2°C
- Evaluation Points: 0 Time, 1 Week, 4 Weeks, 8 Weeks

Beverage Production

- Alternan-oligosaccharide was combined with the flavored waters in a large batch.
- Alternan-oligosaccharide was added to the water while agitating with a Lightning Mixer at 700 rpm. After the entire amount of alternan-oligosaccharide was added, the beverage was mixed for an additional 1.5 minutes.
- The percentages used in the preliminary screening were used for the shelf life. Each variable is present at a level to give a 5 gram per serving level of fiber.
- After mixing, the beverage was aliquoted to separate bottles for storage. The bottles were placed in storage at each temperature.
- One bottle was used for each evaluation point.
- The samples were allowed to come to room temperature before evaluation.

The following protocols were used to evaluate the beverages at each interval in the shelf life study.

Hot Fill Simulation - Abuse Condition:

Some beverage processes call for hot filling. This involves heating the beverage to 82°C, holding for 10 minutes at that temperature, cooling to 65°C and then bottling. The maltose-oligosaccharide beverages were exposed to this heat abuse.

Process

- 500 ml of a 2.60% solution of maltose-oligosaccharide in Metro Mint or Fruit$_2$O water was placed in the top of a double boiler.
- Heated to between 82°C and 87.8°C
- Held for 10 minutes at that temperature
- Cooled liquid to 65.6°C
- Bottled
- Beverages observed for 72 hours. Measured for viscosity, pH, and Brix.

Results:

|  | Viscosity (cp) | pH | Brix | Comments |
|---|---|---|---|---|
| Fruit$_2$O Control | 2.16 | 2.89 | 2.8 | |
| Fruit$_2$O Hot fill | 2.16 | 2.94 | 3.0 | No obvious change in appearance. |
| Metro Mint Control | 2.13 | 3.95 | 2.9 | |
| Metro Mint Hot Fill | 2.19 | 4.17 | 2.9 | No obvious change in appearance. |

The maltose-oligosaccharide was tolerant to the hot fill conditions tested.

5. <u>Heat and acid stability of alternan-oligosaccharide</u>

Material: Maltose-oligosaccharide was produced according to a procedure similar to Ex. 2, except that 30% sucrose and 15% maltose were used and the ethanol precipitation step was omitted. The product was purified by filtration techniques and maltose-oligosaccharide was obtained as a syrup containing about 15 weight-% fructose, 68.8° BRIX (internal reference M2).

The alternan-oligosaccharide contained about 15 weight-% of fructose. For preparation of samples all amounts were calculated in such manner that > 5% pure maltose-oligosaccharide was contained in the final sample.

Samples:

- alternan-oligosaccharide in 0.1M HAc - pH 3
- alternan-oligosaccharide in 0.02M HCl - pH 1.5

The samples were incubated for 10 minutes at temperatures of 20, 40, 60, 80, and 95°C, respectively, 1 hour at 120°C, and then cooled down for 10 min. 100 $\mu$l volumes were taken from each sample and neutralized:

$$0.1\text{M HAc: } 100\mu l + 100\mu l \text{ N-Mix HAc } (450\mu l \text{ 1M NaOH ad 10ml})$$

$$0.02\text{M HCl: } 100\mu l + 100\mu l \text{ N-Mix HCl } (175\mu l \text{ 1M NaOH ad 10ml})$$

For HPAEC-analysis 50$\mu$l of a 1:400 dilution of the neutralized sample were injected (SC-HPAEC).

The following table shows the relative peak areas of alternan-oligosaccharides.

The results show that no degradation of oligosaccharides (DP 3-7) is detectable up to a temperature of 120°C in water (pH 7) and acetic acid (pH 3). In HCl (pH 1.5) no degradation is detectable up to a temperature of 95°C. No increase of glucose was detected (data not shown).

At a temperature of 120°C in HCl degradation was detected, shown by increase of detected glucose (data not shown) and shown by the decrease of relative peak area of DP4 - DP7 (see table).

However, it should be taken into account that the 120°C sample was heated for one hour due to technical reasons (autoclave), which is much longer than for the other samples (10 min).

Results pH and temperature-stability of alternan-oligomer

| Sample Name | rel. area (%) | | | | | | |
|---|---|---|---|---|---|---|---|
| | Panose | DP4-1 | DP4-2 | DP5 | DP6-1 | DP6-2 | DP7 |
| M2 H2O 20°C | 0.257 | 0.066 | 0.313 | 0.270 | 0.023 | 0.052 | 0.020 |
| M2 H2O 40°C | 0.257 | 0.066 | 0.313 | 0.269 | 0.023 | 0.052 | 0.019 |
| M2 H2O 60°C | 0.257 | 0.067 | 0.313 | 0.269 | 0.023 | 0.052 | 0.019 |
| M2 H2O 80°C | 0.260 | 0.057 | 0.317 | 0.271 | 0.023 | 0.052 | 0.019 |
| M2 H2O 95°C | 0.257 | 0.067 | 0.314 | 0.269 | 0.023 | 0.052 | 0.019 |
| M2 H2O 120°C | 0.255 | 0.075 | 0.311 | 0.267 | 0.022 | 0.051 | 0.019 |
| | | | | | | | |
| M2 HAc 20°C | 0.257 | 0.066 | 0.313 | 0.270 | 0.023 | 0.052 | 0.019 |
| M2 HAc 40°C | 0.259 | 0.057 | 0.316 | 0.272 | 0.023 | 0.052 | 0.020 |
| M2 HAc 60°C | 0.257 | 0.066 | 0.314 | 0.269 | 0.023 | 0.052 | 0.019 |
| M2 HAc 80°C | 0.257 | 0.066 | 0.314 | 0.269 | 0.023 | 0.051 | 0.019 |
| M2 HAc 95°C | 0.257 | 0.066 | 0.313 | 0.269 | 0.023 | 0.052 | 0.019 |
| M2 HAc 120°C | 0.260 | 0.065 | 0.314 | 0.268 | 0.022 | 0.051 | 0.019 |
| | | | | | | | |
| M2 HCl 20°C | 0.257 | 0.066 | 0.313 | 0.270 | 0.023 | 0.052 | 0.019 |
| M2 HCl 40°C | 0.256 | 0.066 | 0.314 | 0.269 | 0.023 | 0.052 | 0.019 |
| M2 HCl 60°C | 0.257 | 0.066 | 0.314 | 0.269 | 0.023 | 0.051 | 0.019 |
| M2 HCl 80°C | 0.260 | 0.057 | 0.317 | 0.271 | 0.023 | 0.052 | 0.020 |
| M2 HCl 95°C | 0.260 | 0.066 | 0.312 | 0.268 | 0.023 | 0.052 | 0.019 |
| *M2 HCl 120°C* | 0.397 | *0.055* | *0. 258* | 0.229 | *0.018* | *0.031* | *0.012* |

6. Heat and acid stability of alternan-polysaccharide

Method 1:

| | |
|---|---|
| material: | alternan-polymer was prepared in concentrations of 1%, 5%, 10% or 12% |
| environments: | water (pH 6-7), acetic acid (pH 3.5-5), orange lemonade (pH 3) or hydrochloric acid (pH 1.3-1.5) |
| temperatures: | 40°C, 60°C, 80°C, 95°C, in autoclave only: 120°C |
| incubation times: | 0.5 h, 1 h, 4 h, 24 h and 2-3 weeks |

Method 2:

| | |
|---|---|
| material: | alternan-polymer was prepared in concentrations of 1.25% and 2.5% in orange juice |
| environments: | orange juice (pH 4) |
| procedure A: | alternan is added to cold orange juice, heating to 62.8 - 65.6°C, pasteurization for 30 min at 62.8°C |
| procedure B: | alternan is added to preheated orange juice, (62.8°C), pasteurization for 30 min at 62.8°C |
| storage in refrigerator | for 1, 3, 7, 10, 20, 30 and 60 days |

Analysis for both methods was done with GPC MALLS. Results are presented in the following table.

The results show that alternan-polymer is stable at pH 6-7 when heated. At pH 3-4 alternan-polymer is stable at room temperature. Slight degradation is detected at pH 3-4 when alternan-polymer is heated at 60°C. Considerable degradation is detected at pH 3-4 when alternan-polymer is heated at 80°C.

Results pH and T-stability of alternan-polymer

| pH | T (°C) | Conc. (%) | incubation | Reduction of molar mass* | degradation %* |
|---|---|---|---|---|---|
| 6-7 | 95 | 1;5;10;12 | 0.5-1 h | - | none |
| 3-4 | 20 - 95 | 5 | 1 h | 20% to 60% | at 60°C slight degradation, strong degradation at 80°C and higher |
| 1-1.5 | 20-95 | 5 | 1 h | 28% to 98% | at 60°C strong degradation, very strong degradation at 80°C and higher |
| | | | | | |
| 4.0 | 62 - 65 | 1.25; 2.5 | 30 min, then stored at 4°C for 1 to 60 days | - | none |
| | | | | | |
| 6-7 | 120 | 1; 5;10 | 20 min | - | none |
| 6-7 | 120 | 1; 5;10 | 1h | 14%* | none* |
| 3-4 | 120 | 5;10 | 1h | 95% | very strong deg radation |
| 1-1.5 | 120 | 5;10 | 1h | not evaluable | - |
| * error is about 14%, therefore degradation can not be evaluated when molar mass is reduced by less than 15% | | | | | |

**Claims**

1. Use of alternan as a heat stable ingredient in a foodstuff.

2. Use of alternan according to claim 1, wherein the foodstuff is subjected to a heating-step at a temperature of 50-150°C.

3. Use according to claim 1 or 2 wherein the alternan is an alternan-polysaccharide.

4. Use according to claim 1 or 2 wherein the alternan is an alternan-oligosaccharide.

5. Use according to claim 4 wherein the alternan-oligosaccharide is produced by the reaction of sucrose with an acceptor molecule in presence of alternansucrase enzyme.

6. Use according to claim 5 wherein the acceptor molecule is selected from maltose, isomaltose, maltitol, (iso)maltot-riose and methyl-$\alpha$-D-glucan.

7.  Use according to one of the preceding claims, wherein the foodstuff is selected from dairy products, ice creams, yogurts, milk, puddings, beverages, beer, sauces, soups, retorted foodstuffs, condiments, canned foodstuffs, especially canned fruits, canned fish, canned vegetables, bakery products, cookies, cake, biscuits, meat products, extrusion products, as snacks and cereals, pasta and ready to serve meals.

8.  Use according to one of the preceding claims, wherein the foodstuff is an acidic foodstuff.

9.  Use according to one of claims 4-6, wherein the foodstuff is an acidic beverage with a pH of 6 or below.

10. Method for manufacturing a foodstuff, comprising the addition of alternan as an ingredient to a foodstuff formulation and a heating step.

11. Method according to claim 10, wherein the heating step is carried out at a temperature of 50-150°C

12. Method according to claim 10 or 11 wherein the alternan is an alternan-polysaccharide.

13. Method according to claim 10 or 11 wherein the alternan is an alternan-oligosaccharide.

14. Method according to claim 13 wherein the alternan-oligosaccharide is produced by the reaction of sucrose with an acceptor molecule in presence of alternansucrase enzyme.

15. Method according to claim 14 wherein the acceptor molecule is selected from maltose, isomaltose, maltitol, (iso) maltotriose and methyl-$\alpha$-D-glucan.

16. Method according to one of claims 10-15, wherein the foodstuff is selected from dairy products, ice creams, yogurts, milk, puddings, beverages, beer, sauces, soups, retorted foodstuffs, condiments, canned foodstuffs, especially canned fruits, canned fish, canned vegetables, bakery products, cookies, cake, biscuits, meat products, extrusion products, as snacks and cereals, pasta and ready to serve meals.

17. Method according to one of claims 10-16, wherein the foodstuff is an acidic foodstuff.

18. Method according to one of claims 13-15, wherein the foodstuff is an acidic beverage with a pH of 6 or below.

19. Foodstuff comprising alternan as ingredient, wherein the foodstuff was subjected to a heating step during its manufacture.

20. Foodstuff according to claim 19, which is selected from dairy products, ice creams, yogurts, milk, puddings, beverages, beer, sauces, soups, retorted foodstuffs, condiments, canned foodstuffs, especially canned fruits, canned fish, canned vegetables, bakery products, cookies, cake, biscuits, meat products, extrusion products, as snacks and cereals, pasta and ready to serve meals.

21. Foodstuff according to claim 19 or 20, which is an acidic foodstuff.

Europäisches
Patentamt

European
Patent Office

Office européen
des brevets

## EUROPEAN SEARCH REPORT

**Application Number**

EP 08 10 2399

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEATHERS TIMOTHY D ET AL: "Characterization of a novel modified alternan." CARBOHYDRATE POLYMERS, vol. 54, no. 1, 1 October 2003 (2003-10-01), pages 107-113, XP004447713 ISSN: 0144-8617 * page 107, paragraphs 1,3 * ----- | 1-21 | INV. A23L1/30 A23L1/308 A23L1/09 A23L2/52 A23L1/054 C12N9/10 C12N9/24 C12P19/04 C12P19/18 |
| A | WO 03/010177 A (NASA [US]) 6 February 2003 (2003-02-06) * claims 6,14-17; figures 9,10 * * page 3, paragraph 3-5 * * page 9, paragraph 1 * ----- | 1-21 | |
| X | BEN HARDIN: "Nonfattening Food Additives - From Sugar?" AGRICULTURAL RESEARCH, SCIENCE AND EDUCATION ADMINISTRATION, WASHINGTON, DC, US, [Online] vol. 47, no. 9, 1 January 1999 (1999-01-01), pages 10-11, XP002486611 ISSN: 0002-161X Retrieved from the Internet: URL:http://genes.pp.ksu.edu/is/AR/archive/sep99/sugar0999.pdf> [retrieved on 2008-07-02] * page 10, last line , paragraph 1 - page 11, line R, paragraph 1 * ----- -/-- | 1-21 | |

| | | | **TECHNICAL FIELDS SEARCHED (IPC)** |
|---|---|---|---|
| | | | A23L C12N C12P |

The present search report has been drawn up for all claims

| Place of search | Date of completion of the search | Examiner |
|---|---|---|
| The Hague | 2 October 2008 | Tallgren, Antti |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**EP 2 098 128 A1**

Europäisches Patentamt

European Patent Office

Office européen des brevets

## EUROPEAN SEARCH REPORT

Application Number

EP 08 10 2399

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/011222 A (TNO [NL]; VAN GEEL-SCHUTTEN GERRITDINA H [NL]; EMEIS JOSEPUS JAN [NL]) 25 January 2007 (2007-01-25) * claim 1 * * page 4, paragraph 15 * * page 6, paragraphs 21,23 * ----- | 1-21 | |
| X | DE 102 09 629 B3 (STERN ENZYM GMBH & CO KG [DE]) 8 January 2004 (2004-01-08) * claims 1,8; examples 1,2 * ----- | 1-8, 10-17, 19-21 | |
| X | WO 2006/062410 A (TNO [NL]; VAN GEEL-SCHUTTEN GERRITDINA H [NL]; HEDDES CORNELIUS EGIDIU) 15 June 2006 (2006-06-15) * page 4, paragraph 15; claim 1 * * page 7, paragraph 24 * ----- | 1-8, 10-17, 19-21 | |
| D,X | WO 2006/088884 A (CARGILL INC [US]; CARLSON TING L [US]; WOO ANTON [US]; ZHENG GUO-HUA []) 24 August 2006 (2006-08-24) * claims 1,2,13,14,21,22; examples 4-8 * * page 1, line 1 - page 2, line 21 * ----- | 1-21 | TECHNICAL FIELDS SEARCHED (IPC) |
| A | WO 03/008618 A (TNO [NL]; VAN GEEL-SCHUTTEN GERRITDINA H [NL]) 30 January 2003 (2003-01-30) * page 1, line 4 - page 2, line 5 * * page 5, paragraph 15 * * page 7, paragraph 25; examples 4,5 * ----- | 1-21 | |
| A | US 2006/141127 A1 (STEPHEN JEANETTE [US] ET AL) 29 June 2006 (2006-06-29) * claim 1; examples 1,2 * ----- | 1-21 | |
| | -/-- | | |

The present search report has been drawn up for all claims

| Place of search | Date of completion of the search | Examiner |
|---|---|---|
| The Hague | 2 October 2008 | Tallgren, Antti |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 08 10 2399

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2007/128559 A (BAYER CROPSCIENCE AG [DE]; MEUSER FRIEDRICH [DE]; BAUER INGO [DE]; HEL) 15 November 2007 (2007-11-15) * claims 1,18,23,31; examples 1,2 * ----- | 1-21 | |
| | | | TECHNICAL FIELDS SEARCHED (IPC) |

The present search report has been drawn up for all claims

| Place of search | Date of completion of the search | Examiner |
|---|---|---|
| The Hague | 2 October 2008 | Tallgren, Antti |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 10 2399

This annex lists the patent family members relating to the patent documents cited in the above-mentioned European search report.
The members are as contained in the European Patent Office EDP file on
The European Patent Office is in no way liable for these particulars which are merely given for the purpose of information.

02-10-2008

| Patent document cited in search report | | Publication date | Patent family member(s) | | Publication date |
|---|---|---|---|---|---|
| WO 03010177 | A | 06-02-2003 | US 2003022319 A1 | | 30-01-2003 |
| | | | US 6479275 B1 | | 12-11-2002 |
| WO 2007011222 | A | 25-01-2007 | NONE | | |
| DE 10209629 | B3 | 08-01-2004 | NONE | | |
| WO 2006062410 | A | 15-06-2006 | NONE | | |
| WO 2006088884 | A | 24-08-2006 | AU 2006214433 A1 | | 24-08-2006 |
| | | | CA 2597886 A1 | | 24-08-2006 |
| | | | EP 1856270 A1 | | 21-11-2007 |
| | | | JP 2008529534 T | | 07-08-2008 |
| WO 03008618 | A | 30-01-2003 | CA 2454563 A1 | | 30-01-2003 |
| | | | JP 2005500839 T | | 13-01-2005 |
| | | | NZ 530638 A | | 27-01-2006 |
| | | | US 2005059633 A1 | | 17-03-2005 |
| US 2006141127 | A1 | 29-06-2006 | CA 2529330 A1 | | 23-06-2006 |
| WO 2007128559 | A | 15-11-2007 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20070275123 A1
- US 5702942 A
- US 5789209 A
- WO 0047727 A
- EP 2008051760 W
- US 7182954 B
- WO 2006088884 A

### Non-patent literature cited in the description

- **G. L. Cote.** *Carbohydrate Polymers,* vol. 19, 249-252
- **Seymour et al.** *Carbohydrate Research,* 1979, vol. 74, 41-62
- **Cote ; Robyt.** *Carbohydrate Research,* 1982, vol. 111, 127-142
- **Gilles ; Joucla.** *Doctoral Dissertation, Ingenier IN-SA,* 2003
- **Arguello-Morales MA ; Remaud-Simeon M ; Pizzut S ; Sarcabal P ; Willemot R ; Monsan P.** Sequence analysis of the gene encoding altemansucrase, a sucrose glucosyltransferase from Leuconostoc mesenteroides NRRL B-1355. *FEMS Microbiol Lett,* 2000, vol. 182, 81-85
- **Degenkolb J ; Takahashi M ; Ellestad GA ; Hillen W.** *Antimicrob. Agents Chemother.,* 1991, vol. 35 (8), 1591-1595
- **Dotto GP ; Horiuchi K ; Zinder ND.** Initiation and termination of phage f1 plusstrand synthesis. *Proc Natl Acad Sci U S A.,* 1982, vol. 79, 7122-7126
- **Horn, U. ; Strittmatter, W. ; Krebber, A. ; Knüpfer, U. ; Kujau, M. ; Wenderoth, R. ; Müller, K. ; Matzku, S. ; Plückthun, A. ; Riesenberg, D.** High volumetric yields of functional dimeric miniantibodies in Escherichia coli, using an optimized expression vector and high-cell-density fermentation under non-limited growth conditins. *Appl. Microbio. Biotechnol.,* 1996, vol. 46, 524-532
- **Jeong SH ; Bae IK ; Lee JH ; Sohn SG ; Kang GH ; Jeon GJ ; Kim YH ; Jeong BC ; Lee SH.** Molecular Characterization of Extended-Spectrum Beta-Lactamases Produced by Clinical Isolates of Klebsiella pneumoniae and Escherichia coli from a Korean Nationwide Survey. *J Clin Microbiol,* 2004, vol. 42, 2902-2906
- **López-Muguía, A. ; Pelenc, V. ; Remaud, M. ; Biton, J.1 ; Michel, J.M. ; Lang, C. ; Paul, F. ; Monsan, P.** Production and purification of altemansucrase, a glucosyltransferase from Leuconostoc mesenteroides NRRL B-1355, for the synthesis of oligoalternan. *Enzyme Microb. Technol.,* 1993, vol. 15, 77-85
- **Schmidt TGM ; Skerra A.** The random peptide library-assisted engineering of a C-terminal affinity peptide, useful for the detection and purification of a functional Ig Fv fragment. *Protein Eng,* 1993, vol. 6, 109-122
- **Skerra, A.** Use of the tetracycline promoter for the tightly regulated production of a murine antibody fragment in Escherichia coli. *Gene,* 1994, vol. 151, 131-135